# FASCICULE DE BREVET EUROPEEN

(11) **EP 3 689 850 B1**
(45) Date de publication et mention de la délivrance du brevet: **08.09.2021**
(21) Numéro de dépôt: 20151607.7
(22) Date de dépôt: 14.01.2020
(51) Int. Cl.: C07C 309/47, C07C 311/48, C09K 19/32, C25B 9/00, H01M 10/056, C09K 19/40

(54) **ELECTROLYTES POUR GENERATEUR ELECTROCHIMIQUE**
ELEKTROLYTE FÜR ELEKTROCHEMISCHEN GENERATOR
ELECTROLYTES FOR ELECTROCHEMICAL GENERATOR

(30) Priorité: 29.01.2019 FR 1900799
(43) Date de publication de la demande: 05.08.2020
(73) Titulaire: Commissariat à l'Energie Atomique et aux Energies Alternatives, 75015 Paris (FR)
(72) Inventeur: PICARD, Lionel, 38054 GRENOBLE CEDEX 09 (FR); BERNARD, Laurent, 38054 GRENOBLE CEDEX 09 (FR); LYONNARD, Sandrine, 38054 GRENOBLE CEDEX 09 (FR); MENDIL, Hakima, 38054 GRENOBLE CEDEX 09 (FR)
(74) Mandataire: Nony

(56) Documents cités:
- EP-A1- 3 353 262

## Description

### Domaine technique

La présente invention concerne de nouveaux composés pouvant être mis en œuvre comme électrolytes, notamment dans des systèmes de génération ou de stockage électrochimique.

De tels électrolytes peuvent être utilisés dans différents systèmes ou dispositifs électrochimiques, notamment dans des batteries au lithium.

### Technique antérieure

D'une manière classique, le principe de fonctionnement d'un générateur électrochimique repose sur l'insertion et le retrait, aussi appelé « désinsertion », d'un ion de métal alcalin ou d'un proton, dans et depuis l'électrode positive, et le dépôt ou l'extraction de cet ion, sur et de l'électrode négative.

Les principaux systèmes utilisent le cation lithium comme espèce ionique de transport. Dans le cas d'un accumulateur au lithium par exemple, le cation lithium extrait de la cathode, lors de la charge de la batterie, vient se déposer sur l'anode, et inversement, il s'extrait de l'anode pour s'intercaler dans la cathode lors de la décharge. Le transport du proton ou du cation alcalin ou alcalino-terreux, en particulier du cation lithium, entre la cathode et l'anode, est assuré par un électrolyte conducteur ionique.

La formulation de l'électrolyte utilisé revêt un caractère essentiel pour les performances du système électrochimique, en particulier lorsque celui-ci est utilisé à des températures très basses ou très élevées. La conductivité ionique de l'électrolyte conditionne notamment l'efficacité du système électrochimique étant donné qu'elle intervient sur la mobilité des ions entre les électrodes positive et négative.

D'autres paramètres interviennent également dans le choix de l'électrolyte mis en œuvre. Il s'agit notamment de sa stabilité thermique, chimique ou électrochimique au sein du système électrochimique, ainsi que des critères économiques, de sécurité et de respect de l'environnement, incluant notamment la toxicité de l'électrolyte.

D'une manière générale, les électrolytes des systèmes électrochimiques se présentent sous forme liquide, gélifiée ou solide.

En ce qui concerne les électrolytes sous forme liquide, les électrolytes conventionnels de générateurs électrochimiques avec un cation métallique de l'une des deux premières colonnes du tableau périodique des éléments, par exemple au lithium, sont composés d'un sel de ce cation dissous dans un milieu organique ou aqueux (classiquement dans des solvants carbonates, acétonitrile pour les batteries au lithium), en présence ou non d'additifs.

En particulier, les électrolytes conventionnels de supercapacité sont composés d'un sel organique (classiquement un sel de tétraéthylammonium tétrafluoroborate Et₄N-BF₄) dissous dans de l'acétonitrile. Leur utilisation en système complet de stockage électrochimique, par exemple dans une batterie Li-ion, nécessite toutefois d'ajouter un séparateur pour assurer une isolation électrique entre les électrodes positive et négative. Néanmoins, même si ces électrolytes présentent de bonnes conductivités ioniques, il demeure des problèmes de sécurité et de coût dans le cadre de la mise en œuvre de solvants organiques (stabilité thermique faible), et de stabilité électrochimique dans le cadre de la mise en œuvre d'un milieu aqueux.

On peut également citer comme électrolyte, la membrane électrolytique des systèmes de générateur électrochimique de type pile à combustible à membrane d'échange de protons, classiquement constituée d'un polymère à chaîne principale carbofluorée et porteur de groupements pendants comportant des fonctions acide sulfonique, tel que le Nafion®. A l'heure actuelle, l'utilisation de ce type de polymères pour la conduction protonique nécessite toutefois de maitriser le taux d'hydratation de la membrane pour obtenir les performances souhaitées. Ce type de polymère est un polymère semi-cristallin, dont seule la partie amorphe présente des propriétés de conduction, la partie cristalline conférant les propriétés mécaniques nécessaires à son bon fonctionnement en système complet.

Il a également été proposé un mélange d'un polystyrène porteur de groupements sulfonyl(trifluorométhylsulfonyl)imide et de POE pour réaliser une membrane d'électrolyte (Meziane et al. Electrochimica Acta, 2011, 57, 14-19). Ces électrolytes polymères présentent néanmoins des conductivités ioniques insuffisantes, de l'ordre de 9,5.10⁻⁶ S.cm⁻¹ à 70 °C. De plus, il n'est pas possible, pour la plupart des domaines d'application actuels, de mettre en œuvre des températures de fonctionnement supérieures à 70 °C.

Pour les systèmes d'électrolyte au lithium, il a également été considéré, l'incorporation d'un sel de lithium bis (trifluorométhane) sulfonimide (LiTFSI) dans l'unité répétitive, conjointement au styrène, pour former du poly(styrène trifluorométhanesulfonylimide de lithium) P(STFSILi). A l'issue de la polymérisation, le poly(électrolyte) possède une architecture copolymère bloc BAB comportant un bloc central «A» de PEO. La conductivité maximale, de l'ordre de 10⁻⁵ S.cm⁻¹, est obtenue à 60 °C avec un polymère comportant 78 % en masse de POE (R Bouchet et al., Nature Materials (2013), 12, 452). L'incorporation d'un groupe fonctionnel en extrémité d'un copolymère à blocs de ce type s'est révélée bénéfique pour sa conductivité (G. Lo et al., ACS Macro Lett. (2013), 2, 990 et H. Jung et al., Macromolecules (2017), 50, 3224).

Dans une autre variante d'électrolytes, des sulfonamides ont également été considérés pour l'élaboration de (co)-solvants pour les batteries lithium-ion. Ces composés possèdent pour l'essentiel une liaison de l'atome de soufre du sulfonamide à des chaînes alkyles fluorées courtes et de l'azote de ce sulfonamide à des fonctions hydrocarbonées simples (D Bresser et al. EP 3 050 872). Pour sa part, la publication H. Ohno et al. (Solid State Ionics (1999), 124, 323) décrit des composés dont le motif sulfonamide est lié via son atome d'azote à un phényle. Ce sel asymétrique peut être en outre connecté via l'atome de soufre de l'anion sulfonamide à un polymère de type PEO (550 g/mol).

Plus récemment, les inventeurs ont caractérisé les performances de conductivité améliorées de dérivés sulfonamides constitués de cycles aromatiques fonctionnalisés d'une part avec un motif sulfonamide et d'autre part une fonction amine (EP 3 353 262). Les structures chimiques explicitement décrites possèdent une fonction amine porteuses de deux chaines alkyles en C₄ à C₁₈ di-hydroxylées et révèlent une conductivité pouvant atteindre jusqu'à 2.10⁻⁷ S.cm⁻¹ à 100°C.

### Exposé de l'invention

Contre toute attente, les inventeurs ont aujourd'hui découvert que des dérivés sulfonamides apparentés à ceux décrits dans ce document EP 3 353 262 se révèlent encore plus intéressants en termes de performances. Ils possèdent une conductivité ionique élevée et préférence un nombre de transport encore plus proche de l'unité.

Ainsi, la présente invention a pour premier objet des molécules cristal liquide ionique répondant à la formule générale (I) 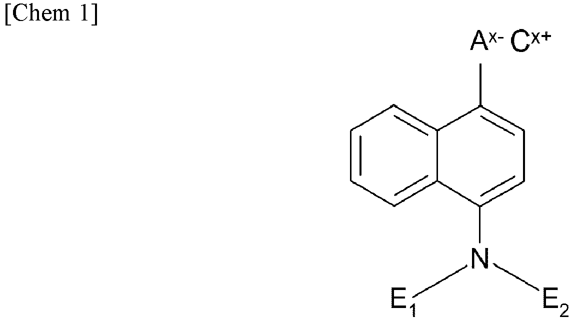 dans laquelle
- E₁ et E₂, identiques ou différents, représentent indépendamment l'un de l'autre un radical hydrocarboné, en C₁₀ à C₁₄, linéaire, saturé et non substitué,
- A^{x-} représente un anion sulfonate ou sulfonylimide de type -SO₂-N⁻-SO₂C_{y}F_{2y+1} avec y entier variant de 0 à 2 et
- C^{x+} représente un contre-cation du groupement anionique -A^{x-}, choisi parmi les ions sodium, lithium et potassium et de préférence est un ion lithium.

Par « cristal liquide ionique », on entend un cristal liquide portant au moins un groupement ionique, à l'image du motif A^{x-}C^{x+} requis selon l'invention.

Le cristal liquide ionique conforme à l'invention est thermotrope.

Il est rappelé qu'un cristal liquide thermotrope se définit par trois types d'états successifs, que celui-ci revêt en fonction de la température. En deçà de sa température de fusion, il est dans un état cristallin (ou phase cristalline). Puis, au-delà de sa température de fusion, il passe dans un état mésomorphe constitué d'une mésophase ou d'une succession de mésophases. Enfin, au-delà de sa température de clarification, il passe dans un état isotrope (ou phase amorphe).

Par « température de fusion », on entend la température à laquelle un cristal liquide thermotrope passe d'un état cristallin à un état mésomorphe.

Par « température de clarification », on entend la température à laquelle un cristal liquide thermotrope quitte sa mésophase ou sa dernière mésophase d'une succession de mésophases pour entrer dans un état isotrope (ou liquide).

Par « état mésomorphe », on entend l'état dans lequel se trouve un cristal liquide thermotrope lorsqu'il est porté à une température supérieure à sa température de fusion et inférieure à sa température de clarification.

Comme illustré dans les exemples qui suivent, les inventeurs ont montré que, lorsque les molécules cristal liquide ionique de l'invention sont dans un état mésomorphe, celles-ci présentent une conductivité ionique pouvant atteindre jusqu'à 3,2.10⁻⁴ S.cm⁻¹ à 100°C soit une valeur supérieure d'au moins un facteur de 1000 comparativement à celles constatées pour les composés décrits dans le document EP 3 353 262.

La plage de température dans laquelle une molécule cristal liquide thermotrope est dans un état mésomorphe peut être déterminée par une méthode connue de l'homme du métier, comme par exemple la DSC (« Differential Scanning Calorimetry » ou calorimétrie différentielle à balayage).

La nature des mésophases d'un état mésomorphe peut être déterminée par une combinaison d'autres caractérisations telles que par MOP (Microscope Optique en lumière Polarisée), par DRX (Diffraction des Rayons X) et/ou par SAXS (« Small Angle X-ray Scattering » ou diffusion des rayons X aux petits angles), cette dernière étant généralement utilisée en complément de la DRX.

Selon un autre de ses aspects, l'invention concerne également l'utilisation d'une molécule cristal liquide ionique thermotrope telle que définie précédemment, dans un état mésomorphe, à titre d'électrolyte dans un système électrochimique.

L'invention concerne encore un électrolyte comprenant, voire étant formé, de molécules cristal liquide ionique thermotrope telles que définies précédemment, dans un état mésomorphe.

Les molécules selon l'invention peuvent être mises en œuvre comme électrolytes dans de nombreux systèmes électrochimiques, tels que les générateurs, par exemple les batteries au lithium.

La mise en œuvre des molécules selon l'invention comme électrolytes s'avère avantageuse à plusieurs titres.

Tout d'abord, ces molécules étant conductrices ioniques dans un état mésomorphe, elles présentent une température d'utilisation comme électrolyte fortement élargie, pouvant être dans toute la gamme de température dans laquelle les molécules sont dans un état mésomorphe, qui correspond généralement à la gamme de température entre la température de fusion et la température de clarification. Les molécules de l'invention peuvent en outre être conductrices ioniques à une température située au-delà de leur température de clarification.

Un système électrochimique, par exemple une batterie au lithium, réalisé à partir d'un électrolyte selon l'invention, peut ainsi fonctionner sur une large plage de température, de préférence entre -60 °C et +300 °C, et plus préférentiellement entre -20 °C et +200 °C.

Par ailleurs, la conductivité ionique d'un électrolyte selon l'invention est basée sur un mécanisme de conduction « direct », par « saut » (« hopping » en langue anglaise) des cations C^{x+} du groupement anionique A^{x-} à l'autre, et non sur un mécanisme assisté comme c'est le cas par exemple des électrolytes polymères proposés par Cohen et al. Molecular Transport in Liquids and Glasses, J. Chem. Phys. 31, 1164 (1959).

Comme il ressort des exemples ci-après, un électrolyte selon l'invention conduit ainsi à des performances significativement améliorées en termes de conductivité ionique.

D'autres caractéristiques, variantes et avantages des molécules et électrolytes selon l'invention, de leur préparation et de leur mise en œuvre ressortiront mieux à la lecture de la description, des exemples et figures qui vont suivre, donnés à titre illustratif et non limitatif de l'invention.

Dans la suite du texte, les expressions « compris entre ... et ... », « allant de ... à ... » et « variant de ... à ... » sont équivalentes et entendent signifier que les bornes sont incluses, sauf mention contraire.

Sauf indication contraire, l'expression « comportant/comprenant un(e) » doit être comprise comme « comportant/comprenant au moins un(e) ».

### MOLECULES DE L'INVENTION

Comme évoqué précédemment, les molécules cristal liquide ionique thermotrope selon l'invention répondent à la formule générale (I)

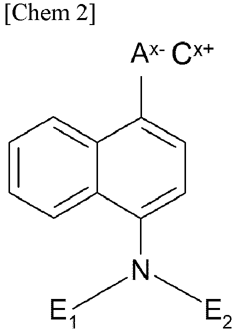

Dans laquelle E₁ et E₂, identiques ou différents, représentent indépendamment l'un de l'autre un radical hydrocarboné, en C₁₀ à C₁₄, linéaire, saturé et non substitué,
A^{x-} représente l'anion sulfonate ou, un anion sulfonylimide de type -SO₂-N-SO₂C_{y}F_{2y+1} avec y entier variant de 0 à 2 et
C^{x+} représente un contre-cation du groupement anionique -A^{x-}, choisi parmi les cations sodium, lithium et potassium et de préférence lithium.

Dans le cadre de l'invention, les radicaux hydrocarbonés figurés par E₁ et E₂ sont généralement identiques et de préférence choisis parmi les radicaux alkyles linéaires non substitués tels que décyle, undécyle, dodécyle, tridécyle et tétradécyle.

En particulier, le radical hydrocarboné figuré par E₁ ou E₂ est en C₁₁ à C₁₃ et plus préférentiellement est le dodécyle.

Selon un mode de réalisation A^{x-} est l'anion sulfonate.

Selon un autre mode de réalisation, -A^{x-} est l'anion de formule -SO₂-N⁻-SO₂-CF₃.

Selon une autre variante de réalisation de l'invention, C^{x+} représente le cation Li⁺.

Selon cette variante, le groupement -A^{x-}c^{x+} représente de préférence un groupe -SO₃⁻Li⁺.

Comme détaillé dans la suite du texte, de telles molécules peuvent être avantageusement mises en œuvre à titre d'électrolyte dans une batterie au lithium.

De préférence, les molécules cristal liquide ionique thermotrope conformes à l'invention ne sont pas mises en œuvre sous la forme de polymères.

Ainsi, les molécules cristal liquide ionique thermotrope conformes à l'invention présentent une masse moléculaire inférieure ou égale à 1 500 g/mol, préférentiellement inférieure à 1 000 g/mol.

La présente invention a notamment pour objet les molécules cristal liquide ionique thermotrope suivantes, particulièrement appropriées à une utilisation comme électrolyte :

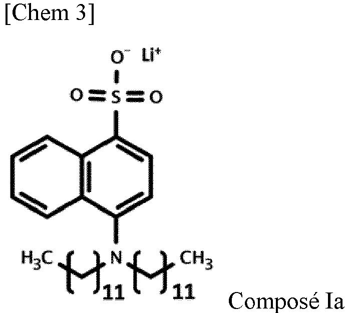

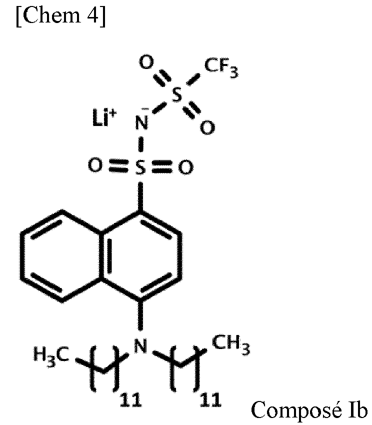

### Préparation des composés de l'invention

Les molécules selon l'invention peuvent être préparées en mettant en œuvre des méthodes de substitution ou d'addition nucléophile connues de l'homme du métier, comme détaillé ci-dessous.

Les molécules de l'invention peuvent être préparées en mettant en présence, dans des conditions propices à leur interaction selon une réaction de substitution ou d'addition nucléophile connues de l'homme du métier, un précurseur du motif naphtalène et un précurseur de la fonction dialkylamine. Cette synthèse peut notamment être réalisée en considérant les protocoles décrits dans le document EP 3 353 262 et les procédés de préparation détaillés ci-après pour les composés la et Ib.

### Utilisation comme électrolyte

Les molécules cristal liquide ionique thermotrope selon l'invention peuvent avantageusement être utilisées, dans un état mésomorphe, à titre d'électrolyte dans un système électrochimique.

Comme mentionné ci-dessus, un état mésomorphe désigne la mésophase ou la succession de mésophases dans laquelle les molécules cristal liquide ionique thermotrope selon l'invention se trouvent en fonction de leur température, située entre la température de fusion et la température de clarification.

L'électrolyte formé de telles molécules est avantageusement mis en œuvre en association avec un séparateur poreux sur lequel ledit électrolyte est imprégné, ledit séparateur assurant une séparation physique entre les deux électrodes du système électrochimique.

A titre de séparateur, on peut utiliser tout séparateur poreux conventionnellement utilisé dans un système électrochimique, comme par exemple un séparateur poreux de batterie au lithium ou une membrane échangeuse d'ions d'une pile à combustible. L'homme du métier est en mesure de choisir un séparateur adapté à la mise en œuvre de l'électrolyte.

Les molécules cristal liquide ionique thermotrope selon l'invention dans lesquelles C^{x+} représente un cation Li⁺ peuvent avantageusement être utilisées, dans un état mésomorphe, à titre d'électrolyte dans une batterie au lithium.

### Electrolyte

Ainsi, selon un autre de ses aspects, la présente invention se rapporte à un électrolyte comprenant, voire étant formé, de molécules cristal liquide ionique thermotrope telles que définies ci-dessus, dans un état mésomorphe.

Dans l'électrolyte de l'invention, les molécules cristal liquide ionique thermotrope sont de préférence mises en œuvre à une température comprise de 80 °C à 220 °C, généralement comprise de 100 °C à 200 °C, préférentiellement comprise de 130 °C à 170 °C, par exemple de l'ordre de 150 °C.

De préférence, l'électrolyte liquide de l'invention présente une viscosité supérieure ou égale à 10 mPa.s, de préférence comprise de 100 mPa.s à 100 Pa.s, à une température comprise entre -60 °C et 300 °C.

Par « à une température comprise entre -60 °C et 300 °C », on entend que l'électrolyte liquide de l'invention présente une viscosité telle que définie ci-dessus à au moins une température située dans cette plage. Ceci ne signifie pas nécessairement que l'électrolyte liquide de l'invention présente une viscosité telle que définie ci-dessus à toute température située dans cette plage.

La viscosité peut être mesurée par extrapolation à cisaillement nul de la courbe de viscosité en fonction du gradient de cisaillement à une température donnée, mesurée sur un viscosimètre/rhéomètre cône/plan ou plan/plan.

Cette condition sur la viscosité de l'électrolyte liquide assure une bonne imprégnation de celui-ci dans le séparateur du système électrochimique.

L'électrolyte selon l'invention présente de bonnes propriétés de conductivité ionique.

En particulier, l'électrolyte de l'invention présente avantageusement une conductivité ionique à 20 °C supérieure ou égale à 10⁻⁷ S.cm⁻¹. En particulier, un électrolyte selon l'invention peut avantageusement posséder une conductivité ionique supérieure à 10⁻⁴ S.cm⁻¹ à 100°C et une conductivité ionique à 150 °C supérieure ou égale à 10⁻³ S.cm⁻¹.

La conductivité ionique peut être mesurée par spectroscopie d'impédance électrochimique en tension ou en courant, d'après une méthode connue de l'homme du métier.

### Système électrochimique

L'électrolyte selon l'invention peut être mis en œuvre dans un système électrochimique, par exemple pour une batterie au lithium.

La présente invention concerne ainsi, selon encore un autre de ses aspects, un système électrochimique comprenant un électrolyte selon l'invention.

Dans le système électrochimique de l'invention, l'électrolyte est de préférence imprégné sur un séparateur poreux tel que décrit précédemment.

Le système électrochimique peut être un système générateur, convertisseur ou de stockage électrochimique.

Il peut s'agir plus particulièrement d'une batterie primaire ou secondaire, par exemple une batterie au lithium, sodium ou potassium ; un accumulateur lithium-air ou lithium-soufre.

Selon un mode de réalisation particulier, l'électrolyte est mis en œuvre dans une batterie, en particulier une batterie au lithium.

La présente invention concerne également, selon encore un autre de ses aspects, un séparateur poreux imprégné d'un électrolyte selon l'invention.

Un tel séparateur poreux est particulièrement adapté à sa mise en œuvre dans un système électrochimique tel que décrit ci-dessus.

L'invention va maintenant être décrite au moyen des exemples et figures suivants, donnés bien entendu à titre illustratif et non limitatif de l'invention.

### Brève description des dessins

[Fig 1] Figure 1 : Analyse IRTF du composé la.
[Fig 2] Figure 2 : Analyse calorimétrique du composé la par DSC sous argon et avec une vitesse de chauffage de 10°K/min.
[Fig 3] Figure 3: Analyse du composé la par SAXS.
[Fig 4] Figure 4 : Analyse IRTF du composé Ib.
[Fig 5] Figure 5 : Analyse du composé Ib par SAXS.
[Fig 6] Figure 6: Analyse de conductivité ionique, en montée et en descente de température, des composés la et Ib versus des produits non conformes à l'invention.

### EXEMPLE 1

### Préparation du composé la conforme à l'invention

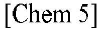

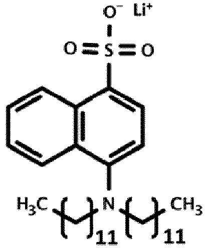

1,25 g d'acide 4-aminonaphtalène sulfonique, 3,50 g de 1-bromododecane et 2,3 mL de triéthylamine sont dissous dans 50 mL de DMF. La solution est agitée à 70°C pendant 48h. 40 mL d'eau permutée et 2 mL de solution aqueuse HCL 2 M y sont ajoutés et la phase aqueuse est extraite 3 fois avec 30 mL de dichlorométhane. Le produit est purifié sur colonne de silice (MeOH/DCM) et les fractions contenant le produit souhaité sont rassemblées et évaporées. 1,74 g de produit bi-substitué est obtenu sous forme de poudre jaunâtre. Suite à une neutralisation par une solution de LiOH diluée, le produit A est obtenu.

### Caractérisation du cristal liquide

*RMN 1H (400 MHz ; MeOD ; 300 K)* : δ ppm 8,7 (d, 1H) ; 8,0 (d, 1H) ; 7,9 (d, 1H) ; 7,5 (dd, 1H) ; 7,4 (dd, 1H) ; 6,5 (d, 1H) ; 3,2 (d, 2H) ; 3,0 (d, 2H) ; 1,4 (m, 40H) ; 0,88 (t, 6H)

*RMN 13C (400 MHz ; MeOD; 300 K)* : δ ppm 131,6 ; 129,0 ; 128,1 ; 127,3 ; 125,4 ; 124,9 ; 122,0 ; 101,6 ; 53,8 ; 44,8 ; 33,1 ; 30,8 ; 30, 7 ; 30,5 ; 29,9 ; 28,5 ; 23,8 ; 22,6 ; 14,4 ; 7,6

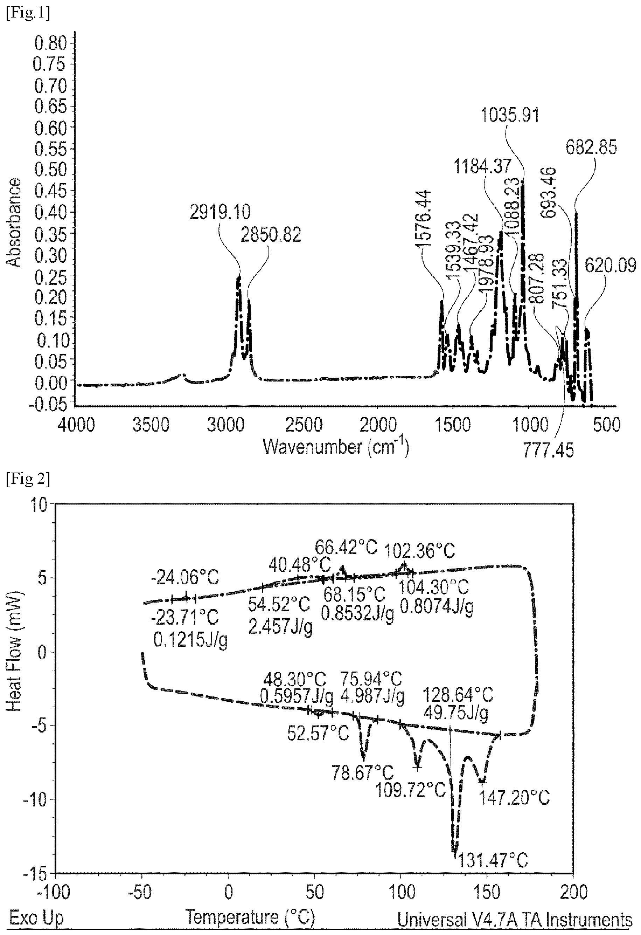

Son spectre 2-FTIR réalisé sur ATR sur un appareil Thermo Scientific Nicolet 6700 est représenté en figure 1.

Le produit la a été caractérisé par DSC sous argon et avec une vitesse de chauffage de 10°K/min. Les résultats de l'analyse calorimétrique sont représentés en figure 2. L'analyse est effectuée avec un gradient en température de 10°C/min sous atmosphère inerte. L'échantillon est chauffé de température ambiante à 180°C au cours d'une première chauffe non représentée en figure 2, puis un cycle refroidissement jusqu'à -50°C suivi d'un second chauffage jusqu'à 180°C est appliqué et les points mesurés sont représentés ci-dessous.

Le produit montre 5 transitions en chauffe à 52°C, 78°C, 109°C, 131,5°C, 147,2°C et 4 en refroidissement à 102°C, 66,4°C, 40,5°C et -24°C.

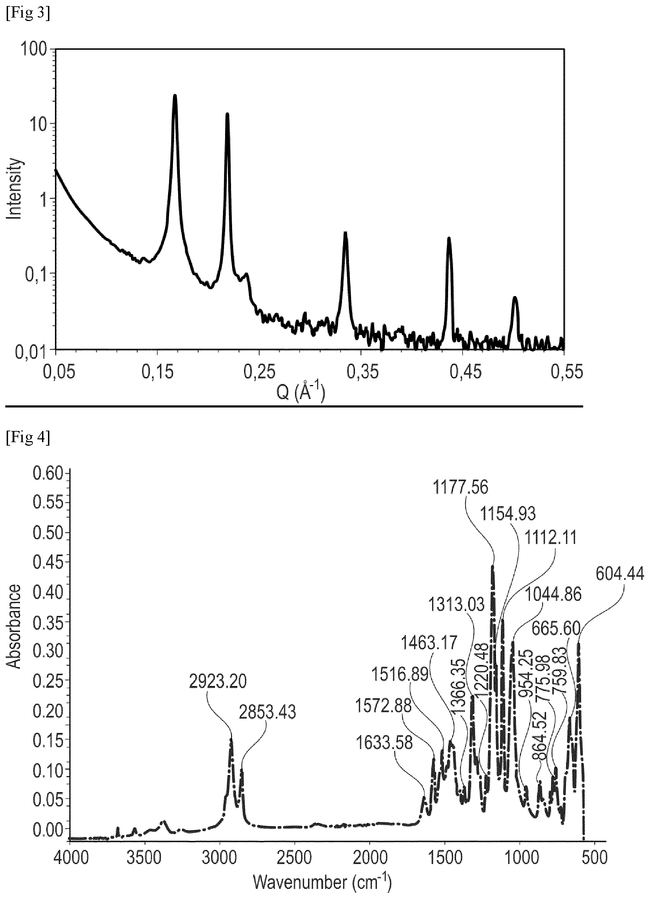

Pour l'analyse SAXS. Un échantillon de poudre de composé la est placé entre deux films de kapton sous atmosphère inerte et mesuré sur une ligne SAXS. Une anode tournante au cuivre et un détecteur Vantec 2000 sont utilisés. Le signal obtenu est un anneau de densité équivalente (donc le matériau est isotrope), après intégration radiale, on obtient le spectre 2D représenté en figure 3. L'état organisé de ce matériau est caractérisé par la présence de pics de Bragg fins et intenses. Le cliché SAXS du produit la correspond à une structure rectangulaire colonnaire à température ambiante.

### EXEMPLE 2

### Synthèse du composé Ib

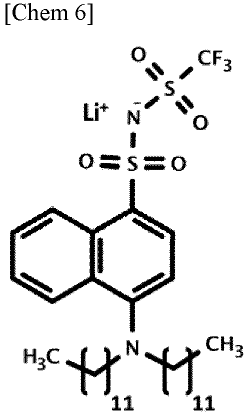

### Synthèse du synthon ANTFSI :

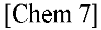

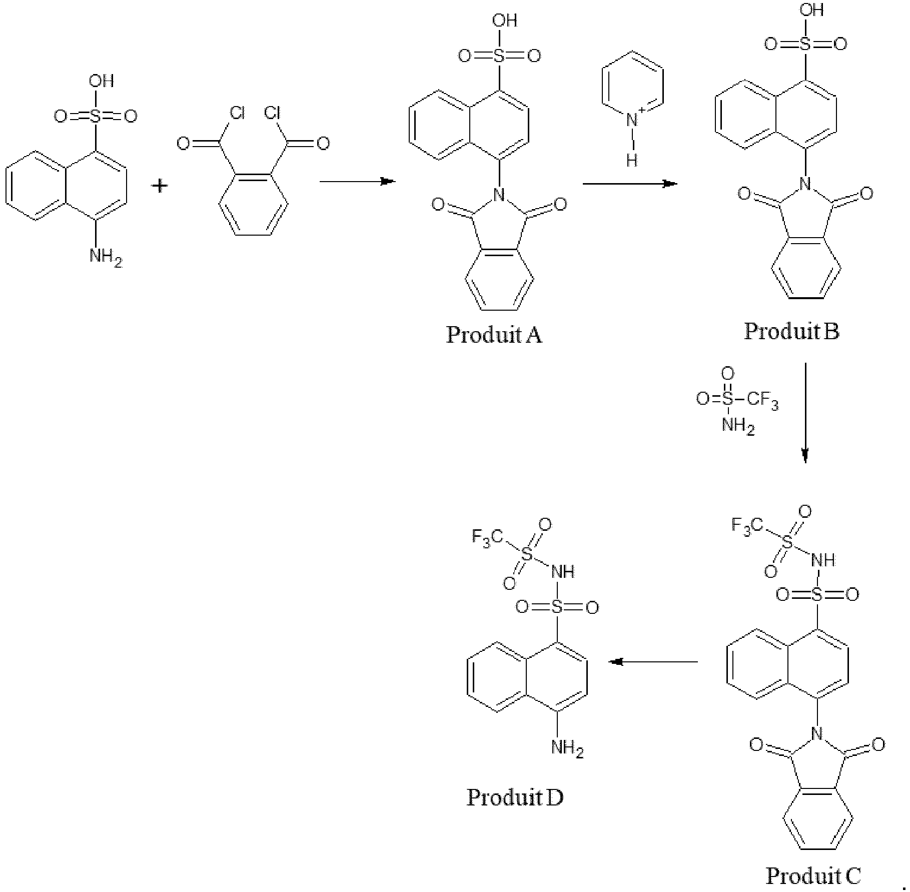

### Etape n°1 :

Dans un ballon monocol de 500mL et sous atmosphère inerte, l'acide Amino-4-Naphtalène sulfonique - ANH (14,97g, 67,13 mmol) est pesé. 100 mL de pyridine sont ajoutés au goutte à goutte sur l'ANH toujours sous atmosphère inerte puis 13 mL (1,3 éq) de chlorure de phtaloyle sont ajoutés sous agitation. Un dégagement de fumée et une coloration ambrée de la solution apparaissent dès l'ajout de la première goutte de chlorure de phtaloyle. Le milieu réactionnel est agité à reflux pendant 24h. La pyridine est évaporée et le résidu est recristallisé à 3 reprises dans du méthanol jusqu'à l'obtention du produit A (21,698g, 75% de rendement après purification).

### Etape n°2 :

Le produit A (21, 698g, 50,290mmol) est ajouté dans 100mL de DMF anhydre dans un ballon de 500 mL tricol inerté sous argon. Une fois le produit solubilisé, le milieu réactionnel est placé dans un bain de glace à 3-5°C et le chlorure de thionyle (7,5mL, 2 éq) est ajouté au goutte à goutte via une ampoule de coulée. A la fin de l'addition, le milieu réactionnel est réchauffé à température ambiante sous agitation. Après 1h de réaction, le milieu réactionnel est versé au goutte à goutte dans de l'eau froide. Le produit 2 est filtré sur Büchner et séché sous vide à 80°C pendant 12h. Le produit B obtenu est une poudre blanche (22,7 g, 98% de rendement).

### Etape n°3 :

Le produit B (22,7g, 61,0 mmol) est ajouté dans 200 mL d'acétone anhydre contenu dans un ballon de 500mL monocol. Puis le trifluoromethanesulfonamide (34,4 g, 2 éq) est ajouté et enfin la triethylamine (20mL, 2,5 éq). Le milieu réactionnel est agité à température ambiante pendant 24h, d'après la Chromatographie sur Couche Mince (CCM) réalisée avec comme éluant le mélange de solvant DCM/MeOH 1:1, la conversion du produit 2 est totale. Le solvant et l'excès de triethylamine sont évaporés sous pression réduite et le résidu sec est purifié sur colonne de silice. Le produit C est obtenu (35,97g, 95% de rendement).

### Etape n°4 :

Le produit C (1g, 2,1 mmol) est placé dans un ballon monocol de 500mL. 300mL d'acétone anhydre sont ajoutés pour dissoudre totalement le produit 3. Puis l'hydrazine (2 éq) est ajoutée au milieu réactionnel qui est par la suite agité pendant 24 h à température ambiante. On note la formation d'un précipité blanc au cours de la réaction. Le solide est ensuite filtré et analysé par RMN 1H pour confirmer sa structure chimique. Le filtrat est évaporé et directement purifié sur colonne de silice. Les fractions contenant le produit souhaité sont rassemblées et le solvant évaporé. La masse de produit D obtenu est 0,51 g soit un rendement de synthèse de 69%.

Le greffage des chaines a été réalisé en deux étapes :

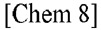

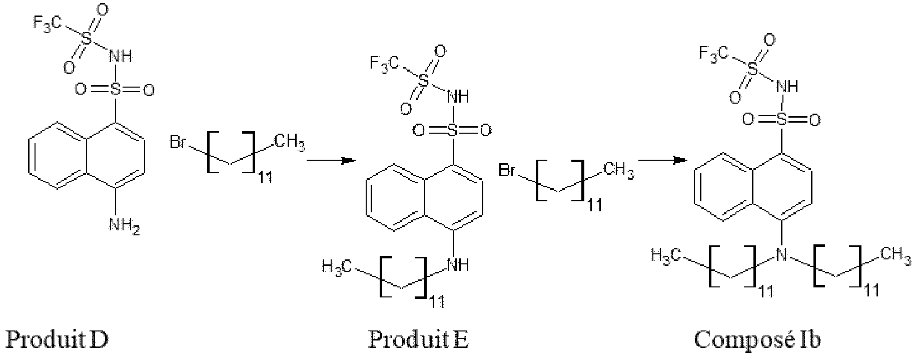

### Etape n°1 :

Dans un ballon de 100 mL sous atmosphère inerte, le produit D (0,500 g, 1,4 mmol) est dissous dans 50 mL de DMF. La triethylamine (0,59 mL, 3 éq) est ajoutée au milieu réactionnel sous agitation. Enfin, du 1-Bromododecane (0,84 mL, 2,5 éq) est ajouté dans la solution. Le milieu réactionnel est agité à 70°C pendant 48h. 40 mL d'eau permutée et 2 mL de solution aqueuse HCL 2M sont ajouté et la phase aqueuse est extraite 3 fois avec 30 mL de dichlorométhane. Le produit est purifié sur colonne de silice (MeOH/DCM) et les fractions contenant le produit souhaité sont rassemblées et évaporées. Le produit E monosubstitué est obtenu sous forme de poudre jaunâtre (0,460 g, 64% de rendement de synthèse).

### Etape n°2 :

Dans un ballon de 100 mL sous atmosphère inerte, le produit E (0,460 g, 0,8 mmol) est dissous dans 50 mL de DMF. La triethylamine (0,37 mL, 3 éq) est ajoutée au milieu réactionnel sous agitation. Enfin, le 1-iodododecane (0,55 mL, 2,5 éq) est ajouté dans la solution. Le milieu réactionnel est agité à 70°C pendant 48 h. 40mL d'eau permutée et 2mL de solution aqueuse HCL 2M sont ajoutés et la phase aqueuse est extraite 3 fois avec 30mL de dichlorométhane. Le produit est purifié sur colonne de silice (MeOH/DCM) et les fractions contenant le produit souhaité sont rassemblées et évaporées. Le produit F est obtenu sous forme de poudre marron (0,503 g, 82 % de rendement de synthèse).

### Caractérisation du cristal liquide

*RMN ¹H (400 MHz ; MeOD ; 300 K)* : δ ppm 8,7 (d, 1H) ; 8,0 (d, 1H) ; 7,9 (d, 1H) ; 7,5 (dd, 1H) ; 7,4 (dd, 1H) ; 6,6 (d, 1H) ; 3,2 (d, 2H) ; 3,0 (d, 2H) ; 1,6 (m, 4H) ; 1,4 (m, 36H) ; 0,88 (t, 6H)

Son spectre 2-FTIR réalisé sur ATR sur un appareil Thermo Scientific Nicolet 6700 est représenté en figure 4.

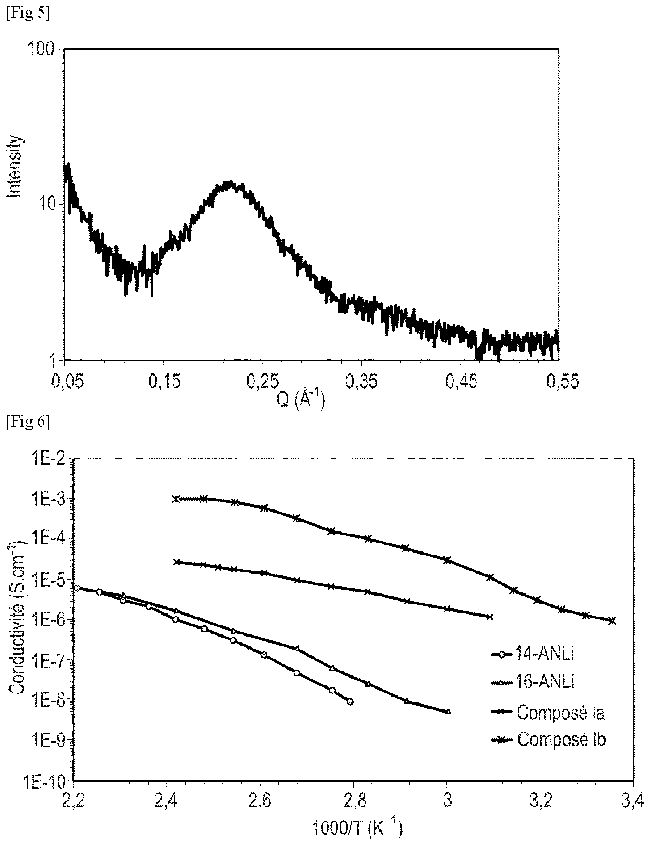

Pour l'analyse SAXS, l'échantillon est placé entre deux films de kapton sous atmosphère inerte et mesuré sur une ligne SAXS. Une anode tournante au cuivre et un détecteur Vantec 2000 sont utilisés. Le signal obtenu est un anneau de densité équivalente (donc le matériau est isotrope), après intégration radiale, on obtient le spectre 2D représenté en figure 5. L'état organisé de ce matériau est caractérisé par la présence de pics de Bragg. Le spectre du produit Ib est caractéristique d'une structure sous forme de lamelles (rapport 1:2).

### EXEMPLE 3

### Caractérisation des conductivités ioniques des composés des exemples 2 et 3

Les mesures de conductivité sont réalisées dans une cellule CESH (Biologic) entre deux électrodes bloquantes. Une différence de potentiel de 50 mV est appliquée entre les deux électrodes bloquantes et un balayage en fréquence entre 5 MHz et 100 mHz est utilisé. Les spectres EIS obtenus sont modélisés par des circuits électriques équivalent (R1 + R2/Q2 + W) pour déterminer la résistance de l'électrolyte.

Les résultats obtenus sont représentés en figure 6 (conductivité ionique en S.cm⁻¹ en fonction de 1000/T, où T est la température en kelvin). A des fins de comparaison, des composés non conformes à l'invention dits 16-ANLi et 14-AN-Li de formules comme suit, 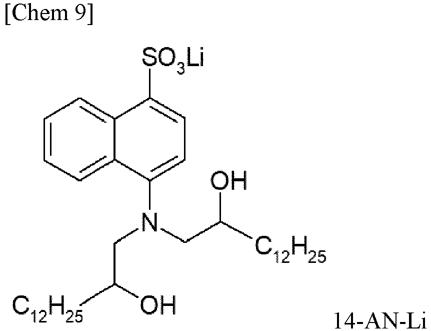 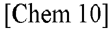 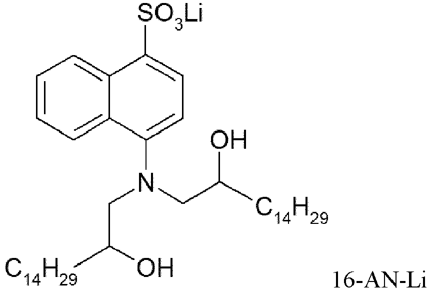 ont été préparés selon les indications figurant dans le document EP 3 353 262 et testés dans les mêmes conditions que les composés la et Ib selon l'invention.

Il apparait que seules, les molécules cristal liquide ionique thermotropes la et Ib conformes à l'invention présentent une conductivité ionique pouvant atteindre jusqu'à 10⁻⁵ S.cm⁻¹ à 103°C, soit 100 fois supérieure à celle mesurée pour les composés non conformes à l'invention. En outre, le composé Ib présente une conductivité jusqu'à un ordre de grandeur supérieure à celle du composé la. Une conductivité de 10⁻⁴ S.cm⁻¹ est en effet obtenue dès 75°C.

Ces résultats démontrent la grande efficacité des molécules cristal liquide ionique thermotropes conformes à l'invention en tant qu'électrolyte dans un système électrochimique, en particulier dans une batterie au lithium.

## Revendications

1. Molécule de cristal liquide ionique thermotrope répondant à la formule générale (I) 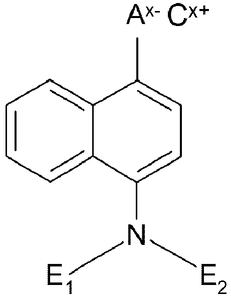 dans laquelle
• E₁ et E₂, identiques ou différents, représentent indépendamment l'un de l'autre un radical hydrocarboné, en C₁₀ à C₁₄, linéaire, saturé et non substitué,
• A^{x-} représente un anion sulfonate ou sulfonylimide de formule -SO₂-N-SO₂C_{y}F_{2y+1} avec y entier variant de 0 à 2 et
• C^{x+} représente un contre-cation du groupement anionique -A^{x-}, choisi parmi les ions sodium, lithium et potassium.

2. Molécule selon la revendication précédente, dans laquelle E₁ et E₂, sont identiques et représentent un radical hydrocarboné, en C₁₁ à C₁₃, et de préférence sont le radical dodécyle.

3. Molécule selon l'une quelconque des revendications précédentes, dans laquelle le groupement anionique -A^{x-} est l'anion sulfonate.

4. Molécule selon l'une quelconque des revendications précédentes, dans laquelle C^{x+} est le cation Li⁺.

5. Molécule selon l'une quelconque des revendications précédentes **caractérisée en ce qu'**elle présente la structure suivante : 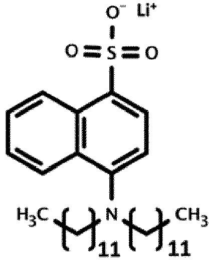 ou

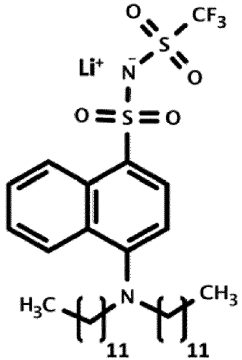

6. Utilisation de molécules cristal liquide ionique thermotrope selon l'une quelconque des revendications 1 à 5, dans un état mésomorphe, à titre d'électrolyte dans un système électrochimique.

7. Utilisation selon la revendication précédente, dans laquelle C^{x+} représente un cation Li⁺, à titre d'électrolyte dans une batterie au lithium.

8. Electrolyte comprenant, voire étant formé, de molécules cristal liquide ionique thermotrope telles que définies selon l'une quelconque des revendications 1 à 5, dans un état mésomorphe.

9. Electrolyte selon la revendication précédente, **caractérisé en ce qu'**il présente une viscosité supérieure ou égale à 10 mPa.s à une température comprise entre -60 °C et 300 °C.

10. Electrolyte selon la revendication 8 ou 9 **caractérisé en ce qu'**il présente une conductivité ionique à 20 °C supérieure ou égale à 10⁻⁷ S.cm⁻¹.

11. Electrolyte selon la revendication 8, 9 ou 10 **caractérisé en ce qu'**il présente une conductivité ionique supérieure à 10⁻⁴ S.cm⁻¹ à 100°C et une conductivité ionique à 150 °C supérieure ou égale à 10⁻³ S.cm⁻¹.

12. Système électrochimique comprenant un électrolyte tel que défini selon l'une quelconque des revendications 8 à 11, ledit électrolyte étant de préférence imprégné sur un séparateur poreux.

13. Système selon la revendication précédente, **caractérisé en ce qu'**il s'agit d'une batterie, en particulier d'une batterie au lithium.

14. Séparateur poreux **caractérisé en ce qu'**il est imprégné d'un électrolyte tel que défini selon l'une quelconque des revendications 8 à 11.

## Patentansprüche

1. Thermotropes ionisches Flüssigkristallmolekül, das der allgemeinen Formel (I) entspricht: 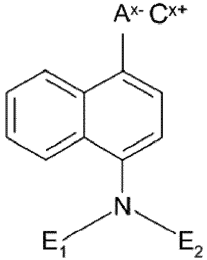 wobei
• E₁ und E₂, die gleich oder verschieden sind, unabhängig voneinander für einen linearen, gesättigten und unsubstituierten C₁₀- bis C₁₄-Kohlenwasserstoffrest stehen,
• A^{x-} für ein Sulfonat-Anion oder Sulfonylimid-Anion der Formel -SO₂-N-SO₂C_{y}F_{2y+1} steht, wobei y von 0 bis 2 variiert, und
• C^{x+} für ein Gegenkation der anionischen Gruppe -A^{x-} steht, das aus Natrium-, Lithium- und Kaliumionen ausgewählt ist.

2. Molekül nach dem vorhergehenden Anspruch, wobei E₁ und E₂ gleich sind und für einen C₁₁- bis C₁₃-Kohlenwasserstoffrest und vorzugsweise für den Dodecylrest stehen.

3. Molekül nach einem der vorhergehenden Ansprüche, wobei es sich bei der anionischen Gruppe -A^{x-} um das Sulfonat-Anion handelt.

4. Molekül nach einem der vorhergehenden Ansprüche, wobei es sich bei C^{x+} um das Li⁺-Kation handelt.

5. Molekül nach einem der vorhergehenden Ansprüche, **dadurch gekennzeichnet, dass** es die folgende Struktur aufweist: 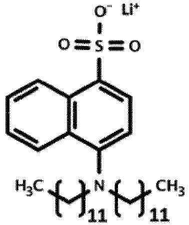 oder

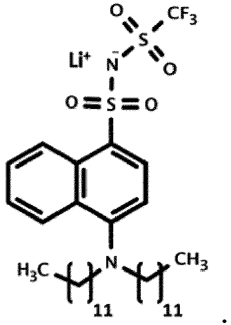

6. Verwendung von thermotropen ionischen Flüssigkristallmolekülen nach einem der Ansprüche 1 bis 5 in mesomorphem Zustand als Elektrolyt in einem elektrochemischen System.

7. Verwendung nach dem vorhergehenden Anspruch, wobei C^{x+} für ein Li⁺-Kation steht, als Elektrolyt in einer Lithiumbatterie.

8. Elektrolyt, der thermotrope ionische Flüssigkristallmoleküle gemäß einem der Ansprüche 1 bis 5 in mesomorphem Zustand umfasst oder sogar daraus gebildet ist.

9. Elektrolyt nach dem vorhergehenden Anspruch, **dadurch gekennzeichnet, dass** er eine Viskosität größer oder gleich 10 mPa.s bei einer Temperatur zwischen -60 °C und 300 °C aufweist.

10. Elektrolyt nach Anspruch 8 oder 9, **dadurch gekennzeichnet, dass** er eine Ionenleitfähigkeit bei 20 °C größer oder gleich 10⁻⁷ S.cm⁻¹ aufweist.

11. Elektrolyt nach Anspruch 8, 9 oder 10, **dadurch gekennzeichnet, dass** er eine Ionenleitfähigkeit größer oder gleich 10⁻⁴ S.cm⁻¹ bei 100 °C und eine Ionenleitfähigkeit bei 150 °C größer oder gleich 10⁻³ S.cm⁻¹ aufweist.

12. Elektrochemisches System, umfassend einen Elektrolyt gemäß einem der Ansprüche 8 bis 11, wobei der Elektrolyt vorzugsweise auf einem porösen Separator imprägniert ist.

13. System nach dem vorhergehenden Anspruch, **dadurch gekennzeichnet, dass** es sich um eine Batterie, insbesondere eine Lithiumbatterie, handelt.

14. Poröser Separator, **dadurch gekennzeichnet, dass** er mit einem Elektrolyt gemäß einem der Ansprüche 8 bis 11 imprägniert ist.

## Claims

1. Thermotropic ionic liquid crystal molecule corresponding to general formula (I) 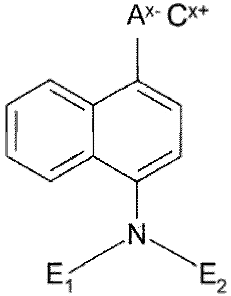 wherein
• E₁ and E₂, which may be identical or different, represent, independently of one another, a linear, saturated and unsubstituted C₁₀ to C₁₄ hydrocarbon-based radical,
• A^{x-} represents a sulfonate anion or a sulfonylimide anion of -SO₂-N⁻-SO₂C_{y}F_{2y+1} with y being an integer ranging from 0 to 2 and
• C^{x+} represents a countercation of the anionic group -A^{x-}, chosen from sodium, lithium and potassium ions.

2. Molecule according to the preceding claim, in which E₁ and E₂ are identical and represent a C₁₁ to C₁₃ hydrocarbon-based radical, and are preferably the dodecyl radical.

3. Molecule according to either one of the preceding claims, in which the anionic group -A^{x-} is the sulfonate anion.

4. Molecule according to any one of the preceding claims, in which C^{x+} is the Li⁺ cation.

5. Molecule according to any one of the preceding claims, **characterized in that** it has the following structure: 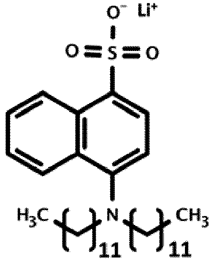 or

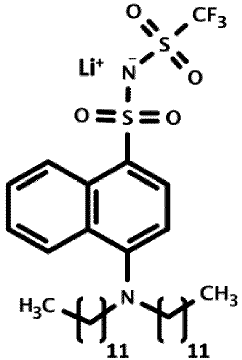

6. Use of thermotropic ionic liquid crystal molecules according to any one of Claims 1 to 5, in a mesomorphic state, as an electrolyte in an electrochemical system.

7. Use according to the preceding claim, in which C^{x+} represents a Li⁺ cation, as an electrolyte in a lithium battery.

8. Electrolyte comprising, or even being formed of, thermotropic ionic liquid crystal molecules as defined in any one of Claims 1 to 5, in a mesomorphic state.

9. Electrolyte according to the preceding claim, **characterized in that** it has a viscosity of greater than or equal to 10 mPa.s at a temperature of between -60°C and 300°C.

10. Electrolyte according to Claim 8 or 9, **characterized in that** it has an ion conductivity at 20°C of greater than or equal to 10⁻⁷ S.cm⁻¹.

11. Electrolyte according to Claim 8, 9 or 10, **characterized in that** it has an ionic conductivity of greater than 10⁻⁴ S.cm⁻¹ at 100°C and an ionic conductivity at 150°C of greater than or equal to 10⁻³ S.cm⁻¹.

12. Electrochemical system comprising an electrolyte as defined according to any one of Claims 8 to 11, said electrolyte preferably being impregnated onto a porous separator.

13. System according to the preceding claim, **characterized in that** it is a battery, in particular a lithium battery.

14. Porous separator **characterized in that** it is impregnated with an electrolyte as defined according to any one of Claims 8 to 11.
